# EUROPEAN PATENT SPECIFICATION

(11) **EP 1 284 756 B1**
(45) Date of publication and mention of the grant of the patent: **15.09.2004**
(21) Application number: 01938245.6
(22) Date of filing: 31.05.2001
(51) Int. Cl.: A61L 27/58, A61L 27/38, A61L 27/18, C12N 5/00, C08L 101/00

(54) **SHAPE MEMORY THERMOPLASTICS AND POLYMER NETWORKS FOR TISSUE ENGINEERING**
MEMORY-THERMOPLASTE UND POLYMERNETZWERKE ZUM GEWEBEAUFBAU
MATIERES THERMOPLASTIQUES A MEMOIRE DE FORME ET RESEAUX POLYMERES POUR GENIE TISSULAIRE

(30) Priority: 31.05.2000 US 208285 P
(43) Date of publication of application: 26.02.2003
(73) Proprietor: Mnemoscience GmbH, 52074 Aachen (DE)
(72) Inventor: LENDLEIN, Andreas, D-52062 Aachen (DE); KNISCHKA, Ralf, 79108 Freiburg i.Br. (DE); KRATZ, Karl, 52070 Aachen (DE)
(74) Representative: Grünecker, Kinkeldey, Stockmair & Schwanhäusser Anwaltssozietät
(86) International application number: PCT/EP2001/006210
(87) International publication number: WO 2001/091822

(56) References cited:
- EP-A- 0 422 693
- WO-A-99/42147
- WO-A-99/42528
- DATABASE WPI Section Ch, Week 199118 Derwent Publications Ltd., London, GB; Class A18, AN 1991-129158 XP002179119 & JP 03 068611 A (DAIKIN KOGYO KK), 25 March 1991 (1991-03-25)

## Description

The present invention relates to the use of shape memory polymers in tissue engineering and to a method of tissue engineering using shape memory polymers.

Up to now biodegradable and biostable thermoplastic or network materials which are biocompatible were used as scaffold material in tissue engineering. Several different polymeric substances were applied from modified biologic to fully synthetic materials. Great achievements have been realized in tissue engineering using these materials, such as tissue engineered artificial skin and other products that are now in the clinical pipeline.

However the scaffold material cannot react or be altered independent of the seeded surface, in-growth of vasculature or differentiation of seeded cells. The materials that are needed are therefore:
Materials or materials loaded or coated with bioactive substances that have the possibility to induce structuring, proliferation or differentiation of cells in itself.
Materials that can control release kinetics of one or more bioactive substances independently by triggering an effect, that may open or close porous structures through an external stimulus.
Scaffolds that will only degrade after an external stimulus is set (degradation on demand).
Scaffolds that can induce forces on the seeded cells.

WO-A-99/4217 and WO-A-99/42528 disclose various SMP materials.

EP-A-0422693 relates to the use of SMP materials in tissue engineering applications.

It is the object of the present invention to overcome the drawbacks of the prior art.

This object has been solved with the use and the method according to the independent claims. Preferred embodiments are given in the dependent subclaims and the claims directed at the method and the polymer scaffold.

### Brief description of the figures

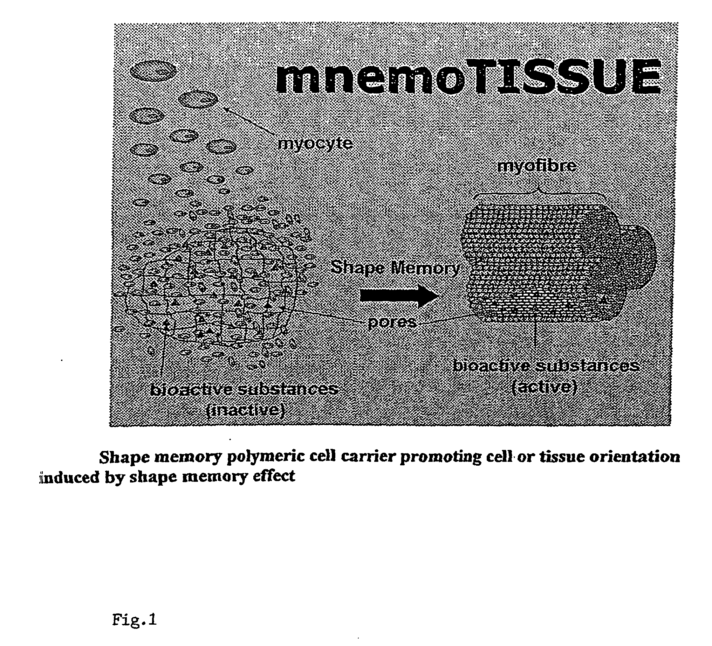

Figure 1 shows SMP particles employed for tissue and/or cell engineering. The surface of the seeded particle can be increased by inducing a shape memory effect.

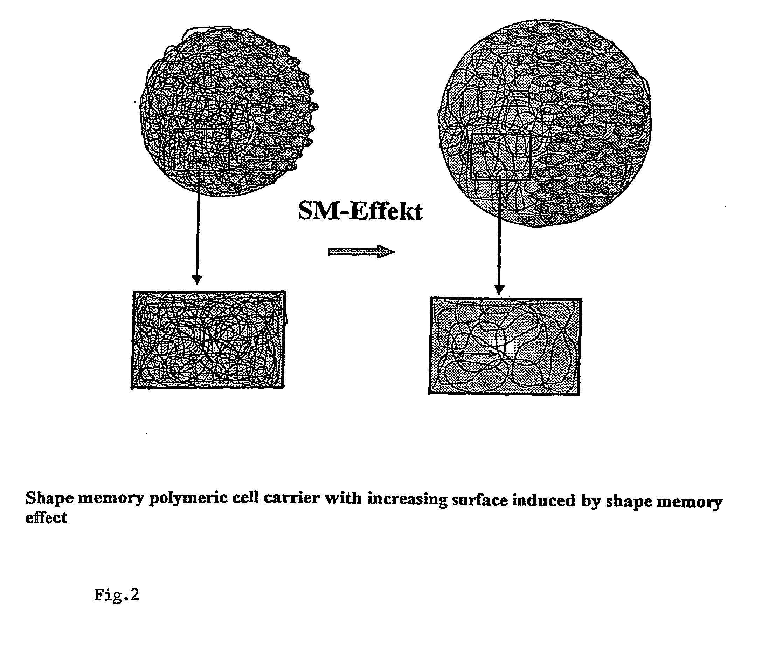

Figure 2 shows that separation of grown cells and/or tissue from SMP particle (scaffold) can be achieved by inducing degradation and/or a shape memory effect.

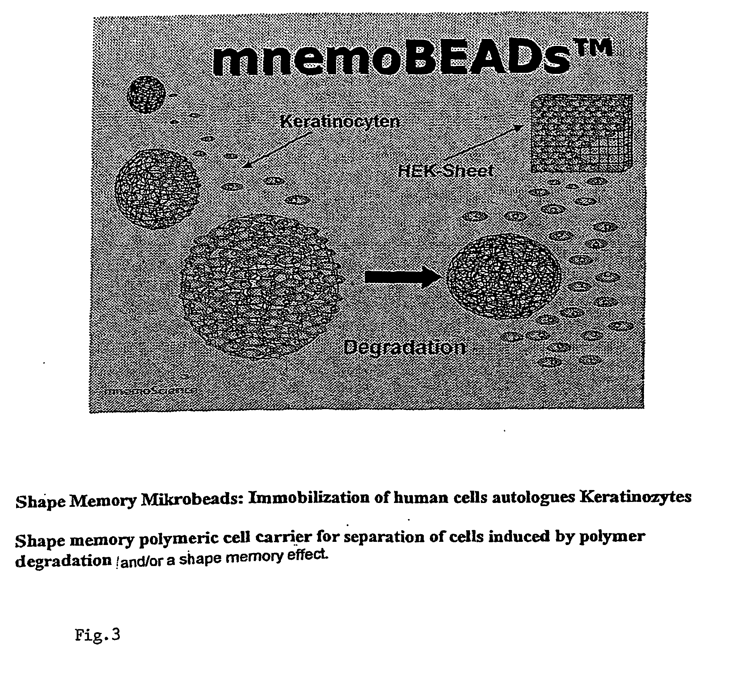

Figure 3 shows an example of the use of SMP scaffold for the orientation of cells and/or tissue grown thereon by inducing a shape memory effect. In addition it is shown that bioactive substances, contained in the SMP scaffold are released due to the shape memory effect.

First the shape memory polymers (SMP) which may be employed in the present invention are described.

SMP are generally characterized as having netpoints and flexible segments. These net-poiints can be chemical or physical in nature. SMP are characterized as phase segregated linear block co-polymers having a hard segment and a soft segment.

Several physical properties of SMPs other than the ability to memorize shape are significantly altered in response to external changes in temperature and stress, particularly at the melting point or glass transition temperature of the soft segment. These properties include the elastic modulus, hardness, flexibility, vapor permeability, damping, index of refraction, and dielectric constant. The elastic modulus (the ratio of the stress in a body to the corresponding strain) of an SMP can change by a factor of up to 200 when heated above the melting point or glass transition temperature of the soft segment. Also, the hardness of the material changes dramatically when the soft segment is at or above its melting point or glass transition temperature. When the material is heated to a temperature above the melting point or glass transition temperature of the soft segment, the damping ability can be up to five times higher than a conventional rubber product. The material can readily recover to its original molded shape following numerous thermal cycles, and can be heated above the melting point of the hard segment and reshaped and cooled to fix a new original shape.

Prefered SMP or SMP compositions can hold more than one shape in memory. For example, the composition can include a hard segment and at least two soft segments. The Tₜᵣₐₙₛ of the hard segment is at least 10 °C, and preferably 20 °C, higher than the Tₜᵣₐₙₛ of one of the soft segments, and the Tₜᵣₐₙₛ of each subsequent soft segment is at least 10 °C, and preferably 20 °C, lower than the Tₜᵣₐₙₛ of the preceding soft segment. A multiblock copolymer with a hard segment with a relatively high Tₜᵣₐₙₛ and a soft segment with a relatively low Tₜᵣₐₙₛ can be mixed or blended with a second multiblock copolymer with a hard segment with a relatively low Tₜᵣₐₙₛ and the same soft segment as that in the first multiblock copolymer. Since the soft segments in both multiblock copolymers are identical,the polymers are miscible in each other when the soft segments are melted. The resulting blend has three transition temperatures: one for the first hard segment, one for the second hard segment, and one for the soft segment. Accordingly, these materials are able to memorize two different shapes.

Articles of manufacture with two or more shapes in memory can be prepared by forming a polymer composition with a hard segment, a first soft segment, and a second soft segment, where the first soft segment has a Tₜᵣₐₙₛ at least 10 °C below that of the hard segment and at least 10 °C above that of the second soft segment. After the composition is shaped at a temperature above the Tₜᵣₐₙₛ of the hard segment, it can be cooled to a temperature below that of the Tₜᵣₐₙₛ of the first soft segment and above that of the second soft segment and formed into a second shape. The composition can be formed into a third shape after it has been cooled below the Tₜᵣₐₙₛ of the second soft segment. The composition can be heated above the Tₜᵣₐₙₛ of the second soft segment to return the composition to the second shape. The composition can be heated above the Tₜᵣₐₙₛ of the first soft segment to return the composition to the first shape. The composition can also be heated above the Tₜᵣₐₙₛ of the hard segment, at which point the composition loses the memory of the first and second shapes and can be reshaped using the method described above.

As used herein, the term "biodegradable" refers to materials that are bioresorbable and/or degrade and/or break down by mechanical degradation upon interaction with a physiological environment into components that are metabolizable or excretable, over a period of time from minutes to three years, preferably less than one year, while maintaining the requisite structural integrity.

A polymer is a shape memory polymer if the original shape of the polymer is recovered by heating it above a shape recovering temperature (defined as the Tₜᵣₐₙₛ of a soft segment) even if the original molded shape of the polymer is destroyed mechanically at a lower temperature than the shape recovering temperature, or if the memorized shape is recoverable by application of another stimulus.

As used herein, the term "segment" refers to a block or sequence of polymer forming part of the shape memory polymer.

As used herein, the terms hard segment and soft segment are relative terms, relating to the Tₜᵣₐₙₛ of the segments. The hard segment(s) has a higher Tₜᵣₐₙₛ than the soft segment(s).

The shape memory polymers can include at least one hard segment and at least one soft segment, or can include at least one kind of soft segment wherein at least one kind of the soft segments are crosslinked, without the presence of a hard segment.

The hard segments can be linear oligomers or polymers, and can be cyclic compounds, such as crown ethers, cyclic di-, tri-, or oligopetides, and cyclic oligo(ester amides).

The physical interaction between hard segments can be based on charge transfer complexes, hydrogen bonds, or other interactions, since some segments have melting temperatures that are higher than the degradation temperature. In these cases, there is no melting or glass transition temperature for the segment. A non-thermal mechanism, such as a solvent, is required to change the segment bonding.

The ratio by weight of the hard segment:soft segments is between about 5:95 and 95:5, preferably between 20:80 and 80:20.

### I. Polymer Segments

The segments preferably are oligomers. As used herein, the term "oligomer" refers to a linear chain molecule having a molecular weight up to 15,000 Da.

The polymers are selected based on the desired glass transition temperature(s) (if at least one segment is amorphous) or the melting point(s) (if at least one segment is crystalline), which in turn is based on the desired applications, taking into consideration the environment of use. Preferably, the number average molecular weight of the polymer block is greater than 400, and is preferably in the range of between 500 and 15,000.

### 1. Thermoset or thermoplastic polymers.

The polymers can be thermoset or thermoplastic polymers, although thermoplastic polymers may be preferred due to their ease of molding.

Preferably, the degree of crystallinity of the polymer or polymeric block(s) is between 3 and 80%, more preferably between 3 and 60%. When the degree of crystallinity is greater than 80% while all soft segments are amorphous, the resulting polymer composition has poor shape memory characteristics.

The polymer segments can be natural or synthetic, although synthetic polymers are preferred. The polymer segments can be biodegradable or non-biodegradable, although the resulting SMP composition is biodegradable biocompatible polymers are particularly preferred for medical applications. In general, these materials degrade by hydrolysis, by exposure to water or enzymes under physiological conditions, by surface erosion, bulk erosion, or a combination thereof. Non-biodegradable polymers used for medical applications preferably do not include aromatic groups, other than those present in naturally occurring amino acids.

Representative natural polymer segments or polymers include proteins such as zein, modified zein, casein, gelatin, gluten, serum albumin, and collagen, and polysaccharides such as alginate, celluloses, dextrans, pullulane, and polyhyaluronic acid, as well as chitin, poly(3-hydroxyalkanoate)s, especially poly(β-hydroxybutyrate), poly(3-hydroxyoctanoate) and poly(3-hydroxyfatty acids).

Representative synthetic polymer blocks include polyphosphazenes, poly(vinyl alcohols), polyamides, polyester amides, poly(amino acid)s, synthetic poly(amino acids), polyanhydrides, polycarbonates, polyacrylates, polyalkylenes, polyacrylamides, polyalkylene glycols, polyalkylene oxides, polyalkylene terephthalates, polyortho esters, polyvinyl ethers, polyvinyl esters, polyvinyl halides, polyvinylpyrrolidone, polyesters, polylactides, polyglycolides, polysiloxanes, polyurethanes and copolymers thereof

Examples of suitable polyacrylates include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate) and poly(octadecyl acrylate).

Synthetically modified natural polymers include cellulose derivatives such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate and cellulose sulfate sodium salt. These are collectively referred to herein as "celluloses".

Representative synthetic degradable polymer segments or polymers include polyhydroxy acids, such as polylactides, polyglycolides and copolymers thereof; poly(ethylene terephthalate); poly(hydroxybutyric acid); poly(hydroxyvaleric acid); poly[lactide-co-(ε-caprolactone)]; poly[glycolide-co-(ε-caprolactone)]; polycarbonates, poly(pseudo amino acids); poly(amino acids); poly(hydroxyalkanoate)s; polyanhydrides; polyortho esters; and blends and copolymers thereof.

Examples of non-biodegradable polymer segments or polymers include ethylene vinyl acetate, poly(meth)acrylic acid, polyamides, polyethylene, polypropylene, polystyrene, polyvinyl chloride, polyvinylphenol, and copolymers and mixtures thereof.

Rapidly bioerodible polymers such as poly(lactide-co-glycolide)s, polyanhydrides, and polyorthoesters, which have carboxylic groups exposed on the external surface as the smooth surface of the polymer erodes, also can be used. In addition, polymers containing labile bonds, such as polyanhydrides and polyesters, are well known for their hydrolytic reactivity. Their hydrolytic degradation rates can generally be altered by simple changes in the polymer backbone and their sequence structure.

Various polymers, such as polyacetylene and polypyrrole, are conducting polymers. These materials are particularly preferred for uses in which electrical conductance is important. Examples of these uses include tissue engineering and any biomedical application where cell growth is to be stimulated. Conducting shape memory polymers are useful in the field of tissue engineering to stimulate the growth of tissue, for example, nerve tissue.

### 2. Hydrogels.

The polymer may be in the form of a hydrogel (typically absorbing up to about 90% by weight of water), and can optionally be ionically crosslinked with multivalent ions or polymers. Ionic crosslinking between soft segments can be used to hold a structure, which, when deformed, can be reformed by breaking the ionic crosslinks between the soft segments. The polymer may also be in the form of a gel in solvents other than water or aqueous solutions. In these polymers, the temporary shape can be fixed by hydrophilic interactions between soft segments.

Hydrogels can be formed from polyethylene glycol, polyethylene oxide, polyvinyl alcohol, polyvinyl pyrrolidone, polyacrylates, poly (ethylene terephthalate), poly(vinyl acetate), and copolymers and blends thereof. Several polymeric segments, for example, acrylic acid, are elastomeric only when the polymer is hydrated and hydrogels are formed. Other polymeric segments, for example, methacrylic acid, are crystalline and capable of melting even when the polymers are not hydrated. Either type of polymeric block can be used, depending on the desired application and conditions of use.

### 3. Polymers Capable of Forming a Gel at Increased Temperatures.

Certain polymers, for example, poly(ethylene oxide-co-propylene oxide) (PLURONICS™), are soluble in water at temperatures lower than body temperature and become hydrogels at temperatures higher than body temperature. Incorporation of these polymers as segments in shape memory polymers provides them with the ability to response to changes in temperature in a manner opposite that of typical shape memory polymers. These materials recover their shape when cooled below their shape recovery temperature, rather than being heated above their shape recovery temperature. This effect is called inversed thermal shape memory effect. Shape memory polymer compositions including these polymer segments are useful in various biomedical applications where the polymer can be inserted as a liquid, and cooled to recover an intended shape *in situ*. The inverse thermal shape memory effect can be obtained by incorporating two different segments into a polymer that are miscible at temperatures lower than T_{misc}, but are immiscible at higher temperatures. The phase separation at higher temperatures stabilizes the temporary shape.

### II. Assembly of Polymer Segments

The shape memory polymer include one or more hard segments and one or more soft segments, wherein at least one of the segments is biodegradable or at least one of the segments is linked to another segment via a biodegradable linkage. Representative biodegradable linkages include ester-, amide-, anhydride-, carbonate-, or orthoester linkages.

### 1. Polymer Structures.

The shape memory effect is based on the polymer morphology. With respect to thermoplastic elastomers, the original shape of an object is fixed by physical crosslinks caused by the hard segment. With respect to network polymers, the soft segments are covalently crosslinked instead of having hard segments. The original shape is set by the crosslinking process.

In contrast to prior art segmented polyurethane SMPs, the segments of the compositions described herein need not be linear. The segments can be partially grafted or attached in dendremeric side groups.

### A. Thermoplastic and Thermoelastic Polymers

The polymers can be in the form of linear dibiock-, triblock-, tetrablock, or multiblock copolymers, branch or graft polymers, thermoplastic elastomers, which contain dendritic structures, and blends thereof. The thermoplastic shape memory polymer composition also can be a blend of one or more homo- or co-polymer with one or more diblock-, triblock-, tetrablock, or multiblock copolymers, branch or graft polymers. These types of polymers are well known to those of skill in the art.

As used herein, the term "degradable thermoset" refers to (i) thermosets SMPs containing only one soft segment, which contains cleavable bonds, and (ii) thermosets containing more than one soft segment, wherein at least one soft segment is degradable or wherein the different soft segments are connected by cleavable bonds. There are four different types of thermoset polymers that have shape memory capability. These include polymer networks, semi-interpenetrating networks, interpenetrating networks, and mixed-interpenetrating networks.

### i. Polymer Networks

A polymer network is prepared by covalently crosslinking macromonomers, i.e., polymers which contain polymerizable endgroups such as carbon-carbon double bonds. The polymerization process can be induced by using light or heat sensitive initiators or by curing with ultraviolet light ("UV-light") without an initiator. Shape memory polymer networks are prepared by crosslinking one or more soft segments which correspond to one or more thermal transitions.

In an embodiment preferred for biomedical applications, the crosslinking is performed using a photocrosslinker and requires no chemical initiator. The photocrosslinker advantageously eliminates the need for initiator molecules, which may be toxic.

### ii. Interpenetrating Networks

Interpenetrating networks ("IPN") are defined as networks where two components are crosslinked, but not to each other. The original shape is determined by the network with the highest crosslink density and the highest mechanical strength. The material has at least two Tₜᵣₐₙₛ corresponding to the different soft segments of both networks.

### iii. Mixed Interpenetrating Network

A mixed IPN includes at least one physically crosslinked polymer network (a thermoplastic polymer) and at least one covalently crosslinked polymer network that cannot be separated by any physical methods. The original shape is set by the covalently crosslinked network. The temporary shapes correspond to the Tₜᵣₐₙₛ of the soft segments and the Tₜᵣₐₙₛ of the hard segment of the thermoplastic elastomer component.

A particularly preferred mixed interpenetrating network is prepared by polymerizing a reactive macromonomer in the presence of a thermoplastic polymer, for example, by the photopolymerization of carbon-carbon double bonds. In this embodiment, the ratio by weight of thermoset polymer to thermoplastic polymer is preferably between 5:95. and 95:5, more preferably, between 20:80 and 80:20.

### iv. Semi-Interpenetrating Networks

Semi-interpenetrating networks ("semi-IPN") are defined as two independent components, where one component is a crosslinked polymer (a polymer network) and the other component is a non-crosslinked polymer (a homopolymer or copolymer), wherein the components cannot be separated by physical methods. The semi-IPN has at least one thermal transition corresponding to the soft segment(s) and the homo- or co-polymer components. The crosslinked polymer preferably constitutes between about 10 and 90% by weight of the semi-interpenetrating network composition.

### v. Polymer Blends

In a preferred embodiment, the shape memory polymer compositions described herein are formed of a biodegradable polymer blend. As used herein, a "biodegradable polymer blend" is a blend having at least one biodegradable polymer.

The shape memory polymers can exist as physical mixtures of thermoplastic polymers. In one embodiment, a shape memory polymer composition can be prepared by interacting or blending two thermoplastic polymers. The polymers can be semicrystalline homopolymers, semicrystalline copolymers, thermoplastic elastomers with linear chains, thermoplastic elastomers with side chains or any kind of dendritic structural elements, and branched copolymers, and these can be blended in any combination thereof

For example, a multiblock copolymer with a hard segment with a relatively high Tₜᵣₐₙₛ and a soft segment with a relatively low Tₜᵣₐₙₛ can be mixed or blended with a second multiblock copolymer with a hard segment with a relatively low Tₜᵣₐₙₛ and the same soft segment as that in the first multiblock copolymer. The soft segments in both multiblock copolymers are identical, so the polymers are miscible in each other when the soft segments are melted. There are three transition temperatures in the resulting blend-that of the first hard segment, that of the second hard segment, and that of the soft segment. Accordingly, these materials are able to memorize two different shapes. The mechanical properties of these polymers can be adjusted by the changing the weight ratio of the two polymers.

Shape memory blends may have better shape memory capabilities than the blend components alone. Shape memory blends are composed of at least one multiblock copolymer and at least one homo- or copolymer. In principle di-, tri, tetra-block copolymers can be used instead of a multiblock copolymer.

Shape memory blends are highly useful in industrial applications, since a broad range of mechanical, thermal, and shape memory capabilities can be obtained from only two or three basic polymers by blending them in different weight ratios. A twin screw extruder is an example of standard process equipment that could be used to mix the components and process the blend.

SMP that may be employed preferably in the present invention are biodegradable and biocompatible SMP, polymer networks as well as thermoplastics, described above, which are employed as scaffold for tissue engineering.

The shape memory effect can be induced thermally, with near infrared, UV, ultrasound or other energy sources. The thermally induced shape memory effect is due to a glass transition or melting point or another thermal effect. Segmented block copoly(ester-urethane)s (thermoplastics) can be used as polymers with physical crosslinks. These copolymers can be used as thermally stimulated shape memory materials with high strain recovery and high final recovery rate. The main advantage of using copolymers is their improved processing conditions as compared with polymers with chemical crosslinks. As only physical crosslinks are introduced, all conventional processing techniques for hermoplastics can be used, and the materials become easily reusable in the case of non-degradable applications.

Thermoset materials with much better mechanical properties e.g. interpenetrating and semi-interpenetrating networks using the same monomers for the macrodiols synthesis but other multifunctional coupling agents e.g. methacrylates can therefore be used and are preferred.

The multiblockcopoly(ester-urethane)s as well as the networks may comprise of coupled cooligomers of ε-caprolactone, L,L-Dilactide, D,L-Dilactide, diglycolide and para-dioxanone. The coupling will be carried out using 2,2(4),4-Trinnethylhexane-diisocyanate in the case of the thermoplastics and using polymerizable methacrylate groups in the case of the thermosets. Synthesis is typically carried out in two steps. In the first step macrodiols with different thermal characteristics are synthesized via ring opening polymerization (ROP) of the cyclic esters and purified. Copolymerization of L-lactic acid with glycolic acid using a tin catalyst for transesterification and ethylene glycol as initiator in a bulk reaction leads to an amorphous soft segment with through the dilactide/diglycolide ratio controlled T_{g}.

To gain multiblockstructures from the synthesized macrodiols, a coupling method must be used. There are several techniques possible from functionalization with a polymerizable end group to direct coupling with a difunctional compound. Coupling of the different segments is e.g. carried out using 2,2(4),4-Trimethyl-hexane-diisocyanate. The synthesis is finished when all isocyanate groups have vanished in the IR-spectrum. It is here very important to use a coupling compound which didn't add an additional crystalline domain to the rather complex multiblock system. The phase separation of the system could be investigated using DSC and AFM. In the case of the thermoset materials the synthesized macrodiols are functionalised using a polymerizable end group as the methacrylate group. The material is melted in the wished form and then cured using e.g. UV light. The shape memory transition temperature can be tailored through different monomer ratios.

One prerequisite for biomedical products is the sterilizability. Several techniques are available for sterilization of medical products as hot sterilization (damp or dry hot air), cold sterilization (ethylene oxide, formaldehyde or ionizing radiation) and aqueous solution sterilization. In the field of sterilization of biodegradable thermoplastics a lot of different techniques are used up to now. They were such as plasma sterilization as well as treatment with ethylene oxide at 50 °C. The use of plasma sterilization is really an interesting idea for the modification of the surface of such materials but not for sterilization of polymers, due to the induced reactions on the surface. Therefore any observed differences in the materials characteristics are difficult to trace back. The same problem exists with the ethylene oxide method at 50 °C. Due to the softening of the implant, ethylene oxide is incorporated into the implant. After the decrease of temperature and ethylene oxide pressure, there is only a slow release of the gas from the material. Toxic reactions of the seeded cells could occur due to the ethylene oxide which is slowly released or the characteristics of the SM material.

### Macrostructures:

Synthetic segmented blockcopoly(ester-urethane)s with thermal shape memory characteristics can be manufactured as woven or non-woven fibres, porous foams or films, membranes, hollow fibers, mono- or multifilaments as well as shape memory thermoset materials processed as thin films or beads.

Processing will be carried out in mono- or multifilament fibres and the formation of 3-dimensional or 2-dimensional structures through different techniques (wovens, non-wovens), formation of films through different techniques such as spin-casting and the formation of3-dimensional porous structures through salt-leaching, thermally induced phase separation, double emulsion technique or gas foaming processes.

The different macroscopic forms could be combined in more sophisticated devices.

These materials can be combined with one or more bioactive substances and cells.

### Bioactive substances:

Representative bioactive substances include growth factors, adhesion proteins, angiogenic factors as well as other compounds.
Growth factors: Epidermal Growth Factor (EGF), Fibroblast Growth Factors (FGFs), Hepatocyte Growth Factor (HGF), Insulin-Like Growth Factor-1 (IGF-1), Insulin-Like Growth Factor-2 (IGF-2), Keratinocyte Growth Factor (KGF), Nerve Growth Factor (NGF), Platelet-Derived Growth Factor (PDGF), Transforming Growth Factor-α (TGF-α), Transforming Growth Factors-β (TGFs-β), Vascular Endothelial Growth Factor (VEGF), recombinant human Growth Hormone (rhGH), angiogenic and anti-angiogenic factors (see, for example, US Patent 6,024,688 (angiogenic factors) Folkman, J.M.; O'Reilly, M. S.; Cao, Y).
Adhesion proteins: RGD, RGDS, GRGDS, cyclic RGD peptides, PHSRN, KQAGDV, LDV, IDAPS, REDV, DGEA, KRLDGS, YIGSR, IKAKV, SIKVAV, CDPGYIGSR-NH2, polylysine, polyomithine, KRSR, RKKRRQRRR, RQK and RNR, VAPG, VGVAPG (Amino acid single letter code).

Preferred bioactive substances are given below:

### Adhesion-promoting oligopeptides.

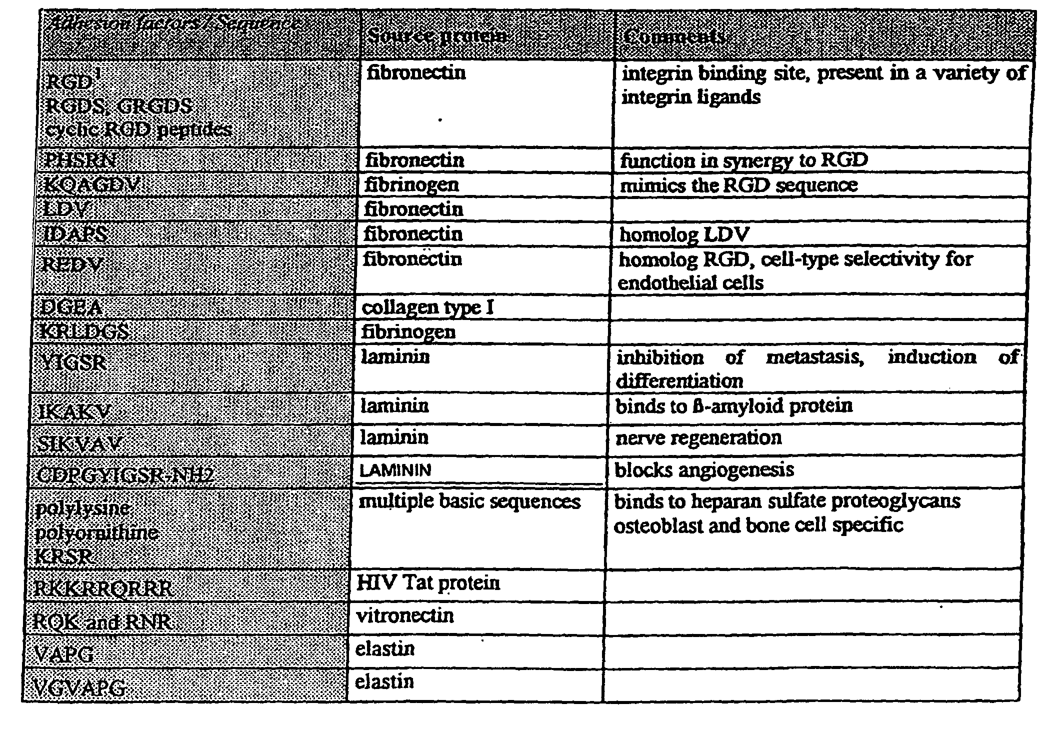

### OVERVIEW GROWTH FACTORS.

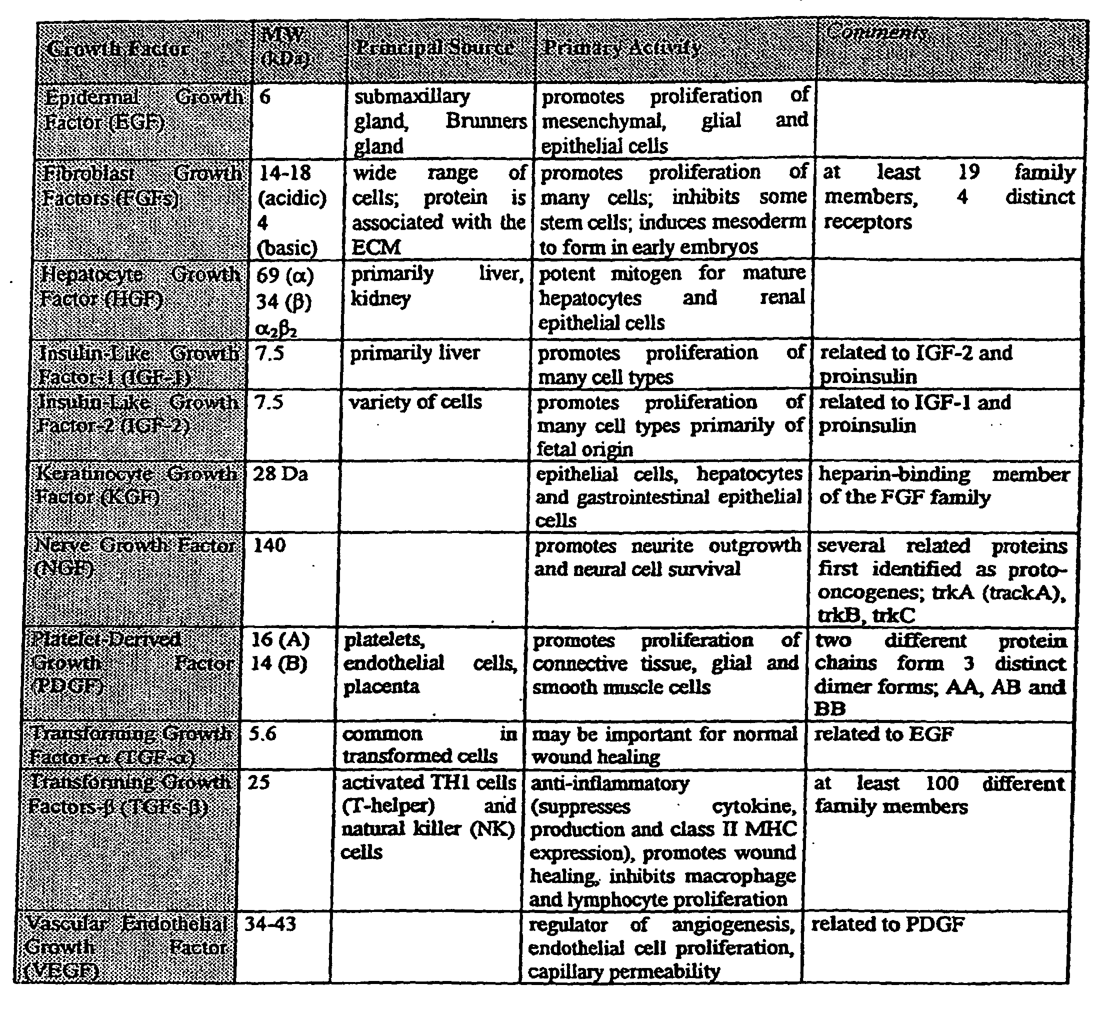

These can be incorporated into the polymeric matrix maintaining their full activity or attached on the surface. The release of one ore more bioactive substances will be controlled using the shape memory effect.

### Cells:

The cells are seeded on the prepared scaffold and taken into a bioreactor for the proliferation of the cells. The shape memory effect is triggered to induce structuring of the cells through applied forces. The bioactive compounds as growth factors, adhesion proteins, angiogenic factors and differentiating factors are released on demand through a triggering of the shape memory effect. Cells to be seeded on the scaffolds are dissociated using standard techniques.
Preferred cell types are mesenchymal adult stem cells, muscle stem cells as well as already differentiated cells as epithelial cells, smooth muscle cells, cardiac muscle cells in co culture, mesothelial cells and chondrocytes, schwann cells, glial cells. In some cases it may also by desirable to include nerve cells. See US Patent 5,869,041 to Vandenburgh relating to muscle cells. For tissue engineered organs where different tissues are needs to be incorporated in one device cocultures of the different cells can be applied.

Cells are preferably autologous cells, obtained by biopsy and expanded in culture for subsequent implantation, although cells from close relatives or other donors of the same species may be used with appropriate immunosupression. After cell expansion within the culture plate, the cells can be easily passaged utilizing the usual technique until an adequate number of cells is achieved.

The shape memory effect will be used to induce forces on the seeded cells. The cells will be seeded on a contracted film. For the structuring of e.g. skeletal muscle, cartilage or nerves the shape memory effect will be triggered and a force is used on the seeded cells that will lead to a alignment in the wished direction.

### Examples of tissue engineered devices:

The prepared shape memory scaffolds will be used for cell seeding. One possibility is the use of muscle cells in combination with the SM polymeric scaffolds.

Muscle transfer is a common procedure in plastic and reconstructive as well as other surgeries but is associated with the risk of morbidity for the donor area. Fabricating skeletal as well as smooth muscle tissue in vitro offers an alternative for this procedure. The key technology for the fabrication of tissue engineered skeletal muscle tissue lies in the alignment and structuring of the cells. Here the new smart shape memory material in combination with attached adhesion proteins may be the material searched for. Two possibilities are shortly outlined here: One possibility is to provide a transplantable internal sphincter muscle. A sterilized biodegradable thermoplastic shape memory elastomer film will be used as seeding surface for smooth muscle cells. The polymer surface may be grafted with adhesion proteins or growth factors that could be released on demand.

Severe heart failure is under the number one death causes in western societies.

As cardiac muscle tissue due not have stem cells, novel approaches for tissue engineered cardiac muscle tissue must be developed. The new smart shape memory materials will provide a scaffold with controllable programmed forces that will affect a co-culture of cardiac muscle tissue with skeletal muscle stem cells. Additional differentiation enhancement may be carried out through in the polymeric scaffold incorporated bioactive substances.

The challenge of ordered integration of a complex arteriovenous and cappilary vascular tree into large living tissue engineered organs ex vivo has still to be solved. For the main aim of providing new organs for transplantation this is an important prerequisite. The loading of a biodegradable thermoplastic shape memory scaffold with angiogenesis factors where the release could be controlled by the shape memory effect is a completely new technique. The reconstruction of whole organs is simply not possible if there is no chance to generate a functioning arteriovenous system in the implant. A further interesting direction is the engineering of larger blood vessels for transplantation using shape memory thermoplasts. Seeding of smooth muscle cells on the outer surface of a shape memory tubing could be used as stabilizing part for blood vessels. The inner surface of such an implant may be covered with epithelial cells to avoid thrombosis.

Preferred SMP that may be employed in the present invention are lactide free SMP. It has surprisingly be found that the lactide free SMP show a degradation behavior differing from the corresponding behavior of lactide containing SMP. While the latter do produce small crystalline particles upon degradation, particles which may present potential health hazard when employed in vivo, the lactide free SMP do not produce small crystalline particles upon degradation. This broadens the possibilities to employ those SMP in tissue engineering, particular to uses in vivo.

A further possibility to alter the SMP employed in the present invention is the provision of coatings on the SMP scaffolds. Such coatings may be employed to further enhance the control of the degradation kinetics or the release of substances, such as the described bioactive substance, from the SMP scaffold during the use in tissue engineering.

Coating of a fast degrading compound with a slow degrading one with low water permeability lead to a degradation on demand. The shape memory effect will be used to alter the diffusion coefficient of water through the slow degrading material or simply destroy the coating through shear forces. The degradation of the coated material will lead to a sharp loss of the structural integrity of the implanted scaffold.

Shape memory polymers can be designed so that the degradation rate is varied. For example, in one embodiment, a hydrolytically degradable polymer can be selectively protected by applying a hydrophobic SMP coating that temporarily prevents water from reaching the hydrolytically cleavable bonds of the bulk polymer. The protective feature of the coating then can be modified when desired by applying an external stimulus such that the diffusion properties of coating are altered to permit water or other aqueous solutions to permeate through the coating and initiate the degradation process. If the hydrolysis rate is relatively high compared to the diffusion rate of water, then the diffusion rate of water through the coating determines the degradation rate. In another embodiment, a hydrophobic coating consisting of densely crosslinked soft segments can be used as a diffusion barrier for water or aqueous solutions. The soft segments should be at least partially crosslinked by linkages that can be cleaved by application of a stimulus. The diffusion rate of water can increased by lowering the crosslinking density.

A further advantage of the method and use according to the present invention is the fact that the SMP may be seeded with cells while the SMP is present in the temporary form. After proliferation of the seeded cells, even in vivo during reconstruction of destroyed tissues, the shape memory effect, i.e. the alteration of the form and/or volume of the SMP scaffold, can be used to exert mechanical forces on the grown tissue in order to induce orientation and/or differentiation, and/or to release bioactive substances contained within the SMP scaffold. This enables for example the orientation of cartilage tissue and/or muscle tissue grown on SMP scaffolds in vivo without the need of invasive surgery.

The SMP scaffolds used in the present invention may be employed in any suitable form, but spheres, pellets, rods, films and tubes are preferred. Usable are also porous materials and foams.

A further advantage of the use and the method of the present invention is the possibility to induce a separation of SMP scaffold and tissue grown thereon using the shape memory effect. This feature can be employed in particular during the tissue engineering of adhesive cells, such as cartilage cells and/or keratinocytes.

For culturing adhesive cells it is necessary that each cell has physical contact to their next neighbours, when the whole surface of the carrier material is occupied by the cells the cell growing stopped and differentiation takes place. So the surface of the carrier material limits the growing of the adhesive cells. Up to now for the immobilization of adhesive cells or bioactive substances, like enzymes, peptides, growth factors or drugs, biocompatible porous glass beads or collagen coated micro carriers based on dextran were used.

SM-microcamers, preferable porous beads, which can be expand by external stimulus are an innovative tool for more efficient way of culturing of adhesive cells. For example. Opening pores or channels by SM-transition in the shell of the microbead causes a controlled continuous swelling of the particle core, that means a continuous increase in the microparticle surface area. This expansion induced by swelling will increase the microparticle surface area in the range of 20% to 500%, preferable from 50% to 200%.

Another difficult step in culturing adhesive cells is the removal of the cells from the carrier material, To remove the cells from the glass or collagen coated microcarrier an aggressive enzyme-cocktail is used, which causes the loss of bioactivity of nearly 20% to 35% of the cells. Therefore the need of an minimal or non invasive removal procedure is obvious. This non invasive removal can also be effected by rapid degradation of the microparticle shell. The degradation kinetics as well as shape memory transition temperature will be tailored using different monomer ratios. Another possibility for minimal invasive removal of the cells is degradation on demand induced by external stimulus.

On the other hand also a second shape memory effect will be used for an easy removal of adhesive cells without loosing their bioactivity, due to drastically changes in the shape of the microcarriers or by switching the surface morphology from smooth to rough or in the different direction. This invention of intelligent shape memory microbeads will lead to more efficient method of culturing every kind of adhesive cells.

Mucosic cells for example can preferably be cultured on shape memory microbeads having a rough surface. Separation can be effected by inducing a shape memory effect changeing the surface morphologie from rough to smooth.

The typical size of the SM-particles is between 10 nanometers and 2,000 microns, preferable 200 nm to 800 µm. The shape of the particles can be spherical, ellipsoidal, cylindrical or random coil shaped, preferable spheres. The microbeads can be solid hard-spheres, or soft-spheres with a determined contend of solvent (gel), or porous material with uniform or gradient polymer density. Also hollow spheres like micells, core-shell particles or bi- or multilayered structures like vesicles for example onions can be used as SM-microbeads.

The SM-microbeads can include eloctroconductive or magnetic particles or particles for diagnostic imaging like radiopaque materials, or biologically active molecules to be delivered or compounds for targeting the microbeads.

These microbeads exhibit an SM-transition with one or more shapes in memory at macroscopic or microscopic lengrthscale induced by external stimulus. The SM-microbeads will consist of biocompatible, biodegradable shape memory polymers that contain at least one physical crosslink (Thermoplast) or contain covalent crosslinks (Thermoset). The shape memory polymers can also be interpenetrating networks or semiinterpenetrating networks.

The used biocompatible, biodegradable shape memory thermoplastic microbeads will be a multiblock copolymer with amorphous and/or crystalline domains consisting of coupled cooligomers of ε-caprolactone, ethylene glycol, propylene glycol, *L,L*-lactic acid, *D,L*-lactic acid, glycolic acid and para-dioxanone.

For the SM-microbeads the use of synthetic segmented blockcopoly(ester-urethane)s with thermal shape memory characteristics as porous foams or films is preferable. These materials will be the porous hydrophobic structural basis of the microparticles. The multiblockcopoly(ester-urethane)s will consist of coupled cooligomers of ε-caprolactone, L,L-lactic acid, D,L-lactic acid, glycolic acid and para-dioxanone. The coupling will be carried out using 2,2(4),4-Trimethylhexane-diisocyanate. Synthesis is typically carried out in two steps. In the first step macrodiols with different thermal characteristics are synthesized via ring opening polymerization (ROP) of the cyclic esters and purified. Copolymerization of L-lactic acid with glycolic acid using a tin catalyst for transesterification and ethylene glycol as initiator in a bulk reaction leads to an amorphous soft segment with through the lactide/glycolide ratio controlled Tg. The oligomerization degree could be tailored using the monomer/initiator-ratio.

For the covalently crosslinked polymernetworks the telecheleic macrodiols werde di- or higher functionalized with any kind of polymerizable endgroups peferable methacrylates or acrylates. The network formation will be induced by radical polymerization, prferable by irridiation with UV-light.

Synthesis of the wished micro carriers relates to processing in the needed form, programming of the shape memory effect and determing of the rate and kinetic of the polymer degradation as also sterilization of the material in a non-invasive manner.

Processing of the needed form means on the one hand the use of emulsion polymerization techniques for the synthesis of the microparticles. On the other hand all conventional methods of crushing and grinding macroscopic material into microparticles can be used, also the use of spraydrying is possible to create the microbeads.

The formation of 3-dimensional porous structures can be induced through salt-leaching, thermally induced phase separation, double emulsion technique or gas foaming processes.

Programming of the shape memory effect means the creation of a permanent structure and shape of the device through heating in a form above the transition temperature of the upper domains and out-balancing of the system. The temporary structure is fixed through heating above the transition temperature of the lower domains, fixation of the temporary structure and quenching. For example through the application of shear stress to the microparticles.

The following synthesis examples illustrate the preparation of SMP materials which may be used in the present invention.

### Thermoplastic SMP with phase segregated block-copolymers

Block-copolymers were produced by connecting macrodiols with diisocyanate.

Synthesis of Telechelics, oligomers with functional groups at both ends.

The telechelic macrodiol were synthesized by ring opening polymerization of cyclic monomers with di(n-butyl)tinoxide as a transesterfication catalyst under a N₂ atmosphere.

α,ω-dihydroxy [oligo(ethylene glycol glycolate) ethylene oligo (ethylene glycol glycolate)] - (PDS 1200 and PDS 1300) was prepared as follows. The monomer p-dioxane-2-one was obtained by distillation (thermal depolymerization) of the oligomer prior to use. 57 g (0.63 mol) of the monomer, 0.673 g (10.9 mmol) ethylene glycol, and 0. 192 g (0.773 mmol) di(n-butyl) tinoxide were heated to 80 °C for 24 h. The end of the reaction (equilibrium) was determined by GPC. The product was soluted in hot 1,2-dichloroethane and filtered hot through a Buechner-funnel filled with silica gel. The product was obtained by precipitation in hexanes and dried in vacuo for 6 h.

α,ω-dihydroxy [oligo(*L*-lactate-*co*-glycolate) ethylene oligo (*L*-lactate-*co*-glycolate)] - (abbr.: PLGA2000-15) was prepared as follows. In a 1000 ml two-neck round bottomed flask, 300 g (2.08 mol) of L,L-dilactide, 45 g (0.34 mol) of diglycolide and 4.94 g (0.80 mol) ethylene glycol were heated to melt at 40 °C and stirred. 0.614 g (2.5 mmol) di(n-butyl) tinoxide was added. After 7 h, the reaction reached equilibrium as determined by GPC. The reaction mixture was soluted in 1,2-dichloroethane and purified in a silica gel column. The product was obtained by precipitation in hexanes and dried *in vacuo* for 6 h.

### Macrodiols of e-caprolactone:

51,5g (0.452 mole) e-caprolactone, 565.6mg (62.1 mmole) ethylene glycole and 373.1mg (1.49 mmole) di-n-butyl-tinoxide were stirred at 135°C for 6 hours.

The reaction mixture was soluted in 1,2-dichloroethane and purified in a silica gel column. The product was obtained by precipitation in hexanes and dried *in vacuo* for 6 h.

### Polyaddition:

The macrodioles were dissolved in dry 1,2-dichloroethane and dried over molecular sieve by azeotropic soxleth distillation. Water content (< 10ppm) was determined in accordance with the Karl Fischer Method. Freshly distilled diisocyanate is introduced into the reaction vessel with a syringe and polymerisation is conducted at 80°C under stirring. The reaction is monitored via GPC. The polymer is obtained by precipitation in hexane, purification is carried out by repeatedly dissolving in 1,2-dichloroethane and precipitating in hexane. The final polymer is dried in vacuum.

These polyadducts comprising caprolactone and dioxane are abbreviated PDC in the following.

The weight-% amount of oligo(*p*-dioxanone) in the polymer is given by the sample ID.

**Table 1.**

| Reaction parameters of PDC multiblock copolymers comprising ODX and OCL. | | | | | | | |
|---|---|---|---|---|---|---|---|
| Sample ID | m (ODX) [g] | n (ODX) [mmol] | m (OCL) [g] | n (OCL) [mmol] | m (TDMI) [g] | n (TDMI) [mmol] | t [h] |
| PDC0 | 0.00 | 0.00 | 5.00 | 1.66 | 0.42 | 1.99 | 45 |
| PDC9 | 0.50 | 0.14 | 4.50 | 1.50 | 0.41 | 1.95 | 45 |
| PDC19 | 1.00 | 0.28 | 4.00 | 1.33 | 0.41 | 1.95 | 45 |
| PDC28 | 1.50 | 0.43 | 3.50 | 1.17 | 0.40 | 1.90 | 45 |
| PDC38 | 12.10 | 3.46 | 17.70 | 5.90 | 2.16 | 10.24 | 504 |
| PDC64 | 3.48 | 0.99 | 1.49 | 0.49 | 0.45 | 2.14 | 45 |
| PDC83 | 5.85 | 1.67 | 0.66 | 0.22 | 0.53 | 2.52 | 45 |

**Table 2.**

| Chemical composition and molecular weights of PDC multiblock copolymers | | | | | | | |
|---|---|---|---|---|---|---|---|
| Sample ID | M_{w} [g·mol⁻¹] | Mₙ [g·mol⁻¹] | M_{w} / Mₙ | Mₙ (Film) [g·mol⁻¹] | OCL [wt.-%] | ODX [wt.-%] | TDMI [wt.-%] |
| PDC0 | 184000 | 77000 | 2.38 | 95000 | 92.3 | 0.0 | 7.7 |
| PDC9 | 159000 | 76000 | 2.10 | 66000 | 83.2 | 9.2 | 7.6 |
| PDC19 | 158000 | 69000 | 2.29 | 61000 | 73.9 | 18.5 | 7.6 |
| PDC28 | 134000 | 63000 | 2.14 | 47000 | 64.8 | 27.8 | 7.4 |
| PDC38 | 118000 | 41000 | 2.87 | | 55.4 | 37.9 | 6.8 |
| PDC64 | 63000 | 35000 | 1.79 | 32000 | 27.5 | 64.2 | 8.3 |
| PDC83 | 65000 | 38000 | 1.72 | 31000 | 9.4 | 83.1 | 7.5 |

The molecular weights of the synthesized materials are measured by relative GPC in chloroform. Calibration was carried out using narrow polystyrene standards.

**Table 5.**

| Selected shape memory properties determined from cyclic thermo-mechanical experiments between - 20 and 50 °C | | | | | | | |
|---|---|---|---|---|---|---|---|
| | | PDC9 | PDC28 | PDC35 | PDC9 | PDC28 | PDC35 |
| N | ε_{prog} [%] | Rᵣ [%] | Rᵣ [%] | Rᵣ [%] | R_{f} [%] | R_{f} [%] | R_{f} [%] |
| 1 | 200 | 76.0 | 79.0 | 80.0 | 99.5 | 99.0 | 98.0 |
| 2 | 200 | 89.0 | 93.5 | 89.5 | 99.5 | 99.0 | 98.0 |
| 3 | 200 | 99.0 | 96.0 | 98.0 | 99.5 | 99.0 | 98.0 |
| 4 | 200 | 99.0 | 99.0 | 99.0 | 99.5 | 99.0 | 98.0 |

Shape memory properties can be measured using cyclic thermo-mechanical tests according to a method described in literature (B. K. Kim, S. Y. Lee, M. Xu, *Polymer* 37, 5781 (1996)). The material is pressed to films having a specific thickness. "Dogbone" shaped samples are punched out of the films and mounted in a tensile tester equipped with a thermo-chamber.

The strain recovery ratio Rᵣ and shape fixity R_{f} can be obtained according to the calculation described in H. Tobushi, H. Hara, E. Yamada, S. Hayashi, *S*.*P*.*I*.*E*. **2716**, 46 (1996). Strain recovery depends on the cycle number N and gradually approaches 100 % after a learning phase during the first couple of cycles. Shape fixity is determined by the amorphous regions able to undergo entropic elasticity after shape programming and remains constant through the different cycles.

The shape memory properties strongly depend on the combination of the two segments. The hard segment is responsible for the mechanical stability of the material, an the soft segment for high strain-recovery ratios.

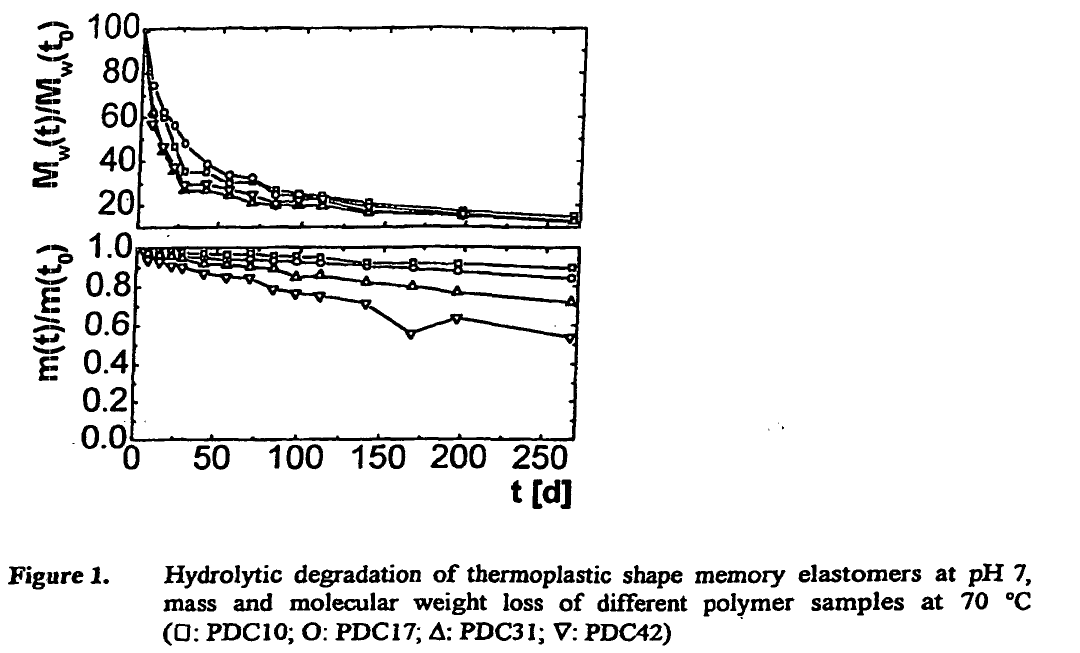

Degradable biomaterials need to contain links that can be cleaved under physiological conditions. There may be enzymatic or hydrolytic scission of the chemical bonds. The hydrolytic cleavage has the advantage that the degradation rate would be independent of the implantation site. Since enzyme concentrations in the body are varying significantly depending on the location, the enzymatically catalyzed cleavage of chemical bonds would be strongly depending on the implantation site. For that reason we introduced hydrolytically cleavable bonds for the synthesis of biodegradable thermoplastic shape memory elastomers. The degradation kinetics could be changed by modification of the used combination of precursor materials. An increase in the amount of hard segment leads to a faster loss in mass as well as molecular weight.

There are several methods to characterize the degradation of a polymer e.g. mechanical properties, molar mass and weight.

The degradation process could be split into 3 stages. In the first stage there is a water uptake and a swelling of the polymer to a considerable amount depending on the hydrophobicity. Some ester linkages are cleaved. In the second stage a considerable loss of the molar mass could be observed. The hydrolysis is auto-catalyzed through acid groups that are built up through the crack of the ester bonds. The mechanical properties are breaking down.

The third stage is characterized through the mass loss of the sample. In some cases highly crystalline polymer particles could be observed at the end of the degradation process. The degradation of poly(L-lactic acid) based materials is leading to the formation of such high crystalline particles. If these particles are to great in size the formation of fibrous capsules in-vivo could be observed. The degradation process is stopped and the encapsulated particles stay in the body and may cause inflammation. The materials show also a non-linear mass loss leading to a sudden release of the degradation products from the bulk material and therefore high concentrations of e.g. lactic acid that may cause inflammation (K. Fu, D.W. Pack , A.M. Klibanov, R. S. Langer *Pharm Res* 17:1, 100, (2000); K.A. Hooper, N.D. Macon, J. Kohn *J*. *Biomed*. *Mat*. *Res.* **32** ,443, (1998). In contrast to these polymers the applied multiblock copolymers are showing a linear mass loss and no formation of crystalline particles. The new smart multiblock shape memory materials show a particle free degradation in in-vitro studies.

### Synthesis of Poly(e-caprolactone) dimethacrylates

The synthesis of poly(e-caprolactone) dimethacrylates (PCLDMA) is performed according to the method described by Aoyagi et al. (19). The procedure is described on the example of PCLDMA2000 (Mw=2000). To a solution of 20 g (10 mmol) poly(e-caprolactone) diol and 5,3 ml triethylamin (38 mmol) in 200 ml of dry THF, 3,7 ml (38 mmol) methachroyl chloride is added dropwise at 0 °C. The solution is now allowed to warm up to room temperature and stirred for 3 days. Then the precipitated salt is filtered off and the solvent is evaporated at room temperature under reduced pressure. The crude product is resolved in 200 ml ethyl acetate, filtered again and precipitated into a ten-fold excess of a mixture of hexanes, ethyl ether and methanol (18:1:1). The colorless precipitate is collected and soluted in 200 ml of dichlorethane, precipitated again and dried carefully under reduced pressure at room temperature. The degree of methacrylation is determined using 1H-NMR-spectroscopy

### Synthesis of the Polymer Networks

A mixture of poly(e-caprolactone) dimethacrylate and the proper amount of n-butylacrylate is heated to 10 °C above the melting temperature (Tm) and filled into a mould formed by two glass plates (25 mm x 75 mm) and a teflon spacer of 0,60 mm thickness. To achieve a good homiogenity, the mould is stored at Tm for another hour. Photocuring is performed with a 100 Watt mercury arc lamp (Ultracure 100ss plus, Efos) on a heated plate at Tm and lasts, unless stated otherwise, 15 min. The distance between lamp head and sample is 5,0 cm. After cooling to room temperature, the isolated weight is determined (miso). The sample is extracted and swollen with a 100-fold excess of dichloromethane overnight, washed carefully and weighted (msw). After drying at room temperature under reduced pressure, the sample is weighted again (md).

**Mechanical and Thermo-Mechanical Experiments:** Tensile tests at room temperature were carried out on an Instron 3100. Experiments at extended temperature and thermocyclic experiments were performed on a Zwick 1410 with a Climatix thermo chamber and an Eurotherm temperature controller. The strain rate was 10 mm/min in all experiments. The thermocyclic program consisted of (a) heating up the sample to Th, extending it to εm, cooling down to Tl in the extended state and (b) unloading to 0 % extension at TI. Th and Tl were held for at least 10 minutes before loading or unloading the sample. Each cycle (a) - (b) was repeated 5 times.

**Tab. 1:**

| Copolymerisates of PCLDMA2000 or PCLDMA10000 and n-butyl acrylate. | | | | | | |
|---|---|---|---|---|---|---|
| | PCLDMA2000 | n-butyl acrylate | | curing temperature | gelation degree | degree of swelling |
| Sample ID^{a} | m [mg] | V [µl] | r_{B}^{b} | T [°C] | G [%] | Q [%] |
| C2 | 300.0 | - | - | 50 | 94 | 410 |
| C2B(11) | 267.9 | 36 | 1 | 50 | 94 | 490 |
| C2B(38) | 187.5 | 148 | 5 | 45 | 92 | 570 |
| C2B(54) | 136.8 | 183 | 10 | 40 | 96 | 660 |
| C2B(65) | 106.5 | 216 | 15 | 40 | 95 | 650 |
| C2B(75) | 76.2 | 250 | 25 | 30 | 92 | 680 |
| C2B(90) | 31.5 | 300 | 75 | 23 | 97 | 730 |
| C10 | 300.0 | - | - | 60 | 90 | 760 |
| C10B(20) | 239.4 | 68 | 10 | 55 | 97 | 800 |
| C10B(39) | 183.7 | 130 | 25 | 50 | 93 | 800 |
| C10B(50) | 149.1 | 169 | 40 | 50 | 90 | 840 |
| C10B(60) | 119.2 | 202 | 60 | 45 | 97 | 880 |
| C10B(71) | 85.0 | 241 | 100 | 40 | 93 | 1020 |
| ^{c} | 57.7 | 271 | 166 | 35 | n.b. | n.b. |

| | | | | | | |
|---|---|---|---|---|---|---|
| ^{a} The two digit number in brackets of the sample ID gives the weight percentage w_{B} of n-butyl acrylate in the monomer mixture. | | | | | | |
| ^{b} r_{B}: molar ratio of comonomers; r_{B} = n_{n-butyl acrylate} / n_{PCLDMA10000} | | | | | | |
| ^{c} No stable film is obtained. Degree of swelling Q of the films: in dichloromethane | | | | | | |

## Claims

1. Use of a shape memory polymer in the manufacture of a tissue engineering scaffold for use in tissue engineering, wherein the shape memory polymer is seeded with dissociated cells.

2. Use in accordance with claim 1, wherein the shape memory polymers are seeded while being in their temporary shape.

3. Use in accordance with claim 1, wherein the shape memory polymers are free of lactide units.

4. Method for in vitro tissue engineering, comprising the seeding of a shape memory polymer with dissociated cells, proliferating the cells and inducing a shape memory effect of the shape memory polymer.

5. The method according to claim 4 wherein the shape memory polymer used is biodegradable.

6. The method according to claim 4 or 5, wherein the shape memory polymer furthermore comprises at least one bioactive material.

7. The method according to any of the preceding claims, wherein the shape memory polymer is free of lactide units.

8. Shape memory polymer scaffold seeded with dissociated cells, **characterised in that** the shape memory polymer scaffold is present in its temporary shape.

9. Shape memory polymer scaffold in accordance with claim 8 having a particle size of from 10 nm to 2000 µm.

10. Shape memory polymer scaffold in accordance with claim 8 or 9 in the form of a sphere, a pellet, a rod, a film, a tube.

11. Shape memory polymer scaffold in accordance with claim 8, 9 or 10, wherein the shape memory effect leads to an alternation of the form and/or volume of the scaffold, an alteration of the surface morphology of the scaffold from smooth to rough or in the different direction, an increase of the surface area of the scaffold.

## Patentansprüche

1. Verwendung eines Formgedächtnis-Polymers in der Herstellung eines Gewebe-Engineering-Gerüsts zur Verwendung beim Gewebe-Engineering, worin das Formgedächtnis-Polymer mit dissoziierten Zellen geimpft ist.

2. Verwendung nach Anspruch 1, worin die Formgedächtnis-Polymere geimpft werden, während sie in ihrer temporären Form sind.

3. Verwendung nach Anspruch 1, worin die Formgedächtnis-Polymere frei von Lactid-Einheiten sind.

4. Verfahren zum *in vitro*-Gewebe-Engineering, umfassend das Impfen eines Formgedächtnis-Polymers mit dissoziierten Zellen, das Vermehren der Zellen und das Induzieren eines Formgedächtnis-Effekts des Formgedächtnis-Polymers.

5. Verfahren nach Anspruch 4, worin das verwendete Formgedächtnis-Polymer biologisch abbaubar ist.

6. Verfahren nach Anspruch 4 oder 5, worin das Formgedächtnis-Polymer weiter wenigstens ein bioaktives Material umfaßt.

7. Verfahren nach einem der vorangehenden Ansprüche, worin das Formgedächtnis-Polymer frei von Lactid-Einheiten ist.

8. Mit dissoziierten Zellen geimpftes Formgedächtnis-Polymer-Gerüst, **dadurch gekennzeichnet, dass** das Formgedächtnis-Polymer-Gerüst in seiner temporären Form vorliegt.

9. Formgedächtnis-Polymer-Gerüst nach Anspruch 8 mit einer Teilchengröße im Bereich von 10 nm bis 2000 µm.

10. Formgedächtnis-Polymer-Gerüst nach Anspruch 8 oder 9 in Form einer Kugel, eines Pellets, eines Stabs, eines Films, eines Röhrchens.

11. Formgedächtnis-Polymer-Gerüst nach Anspruch 8, 9 oder 10, worin der Formgedächtnis-Effekt zu einer Änderung der Form und/oder des Volumens des Gerüsts, einer Änderung der Oberflächen-Morphologie des Gerüsts von glatt nach rau oder in der anderen Richtung, einer Erhöhung der Oberfläche des Gerüsts führt.

## Revendications

1. Utilisation d'un polymère à mémoire de forme dans la fabrication d'une trame d'ingénierie tissulaire dans l'ingénierie tissulaire, où le polymère à mémoire de forme est ensemencé avec des cellules dissociées.

2. Utilisation selon la revendication 1, où les polymères à mémoire de forme sont ensemencés alors qu'ils se trouvent dans leur forme temporaire.

3. Utilisation selon la revendication 1, où les polymères à mémoire de forme sont dépourvus d'unités de lactide.

4. Méthode pour l'ingénierie tissulaire in vitro, comprenant : ensemencer un polymère à mémoire de forme avec des cellules dissociées, faire proliférer les cellules et induire un effet de mémoire de forme du polymère à mémoire de forme.

5. Méthode selon la revendication 4, où le polymère à mémoire de forme utilisé est biodégradable.

6. Méthode selon la revendication 4 ou 5, où le polymère à mémoire de forme comprend de plus au moins une matière bioactive.

7. Méthode selon l'une des revendications précédentes, où le polymère à mémoire de forme est dépourvu d'unités de lactide.

8. Trame de polymère à mémoire de forme ensemencé avec des cellules dissociées, **caractérisée en ce que** la trame de polymère à mémoire de forme est présente dans sa forme temporaire.

9. Trame de polymère à mémoire de forme en accord avec la revendication 8 présentant une taille de particule de 10 nm à 2000 µm.

10. Trame de polymère à mémoire de forme en accord avec la revendication 8 ou 9 en forme de sphère, de granulé, de bâtonnet, de film, de tube.

11. Trame de polymère à mémoire de forme en accord avec la revendication 8, 9 ou 10 l'effet de mèmoire de forme mène à une alteration la forme et/ou du volume de la trame, une alteration de la morphologie de la surface de la trame de lisse à rugueuse ou dans la directio: différente,une augmentation de la surface de la trame.
